Europäisches Patentamt

European Patent Office

Office européen des brevets

(11) Publication number : **0 474 416 A2**

# EUROPEAN PATENT APPLICATION

(21) Application number : 91307797.0

(22) Date of filing : 23.08.91

(51) Int. Cl.⁵ : **C07K 15/14,** C07K 15/04, C12N 5/20, C12P 21/08, G01N 33/577, G01N 33/569

(30) Priority : **24.08.90 GB 9018689**

(43) Date of publication of application :
**11.03.92 Bulletin 92/11**

(84) Designated Contracting States :
**CH DE FR GB LI NL**

(71) Applicant : **DOWELANCO**
**9002 Purdue Road**
**Indianapolis, Indiana 46268-3030 (US)**

(72) Inventor : **Dewey, Frances Mary**
**93 Bagley Wood Road**
**Kennington, Oxford OX1 5NA (GB)**
Inventor : **Priestley, Robert Alan**
**101 Pheasant Walk**
**Littlemore, Oxford OX4 4XX (GB)**
Inventor : **Sturz, Anthony V.**
**P.O. Box 112**
**Winsloe, Prince Edward Island C0A 2HO (CA)**

(74) Representative : **Raynor, John et al**
**W.H. Beck, Greener & Co 7 Stone Buildings**
**Lincoln's Inn**
**London WC2A 3SZ (GB)**

(54) Antigenic material, antibodies raised thereto, hybridomas for producing antibodies, and immunoassays incorporating such antibodies.

㊼    An immunogenic material for raising antibodies to Pseudocercosporella herpotrichoides, which consists essentially of P. herpotrichoides-derived proteinaceous material or glycoprotein which is substantially free of material having a molecular weight of less than 50 kD and of material having a molecular weight of greater than 300 kD. The immunogenic material may be prepared by removing from herpotrichoides-derived material

a) the material which is caused to precipitate by the presence in an aqueous solution thereof of 30 weight percent $(NH_4)_2SO_4$, and

b) the molecular weight fraction which is not caused to precipitate by the presence in an aqueous solution thereof of 70 weight percent $(NH_4)_2SO_4$.

The material may be used to raise antibodies to P. herpotrichoides, by immunizing an animal, and collecting antiserum produced. Cell-lines producing species-specific monoclonal antibodies may be produced by fusing immunocompetent cells thus produced with a myeloma to produce a hybridoma, and selecting hybridoma cell lines producing monoclonal antibodies which do not cross react with fungi other than Pseudocercosporella herpotrichoides.

The monoclonal antibodies produced may be used in immunoassay test kits for P. herpotrichoides-derived material.

EP 0 474 416 A2

This invention relates generally to fungal diseases of plants, and in particular to the fungal disease of cereals commonly known as "Eyespot", caused by Pseudocercosporella herpotrichoides (hereinafter referred to as P. herpotrichoides). The invention includes within its scope antibodies, including monoclonal antibodies raised against P. herpotrichoides, and methods of testing, specifically immunoassays, for P. herpotrichoides.

The eyespot pathogen of cereals, Pseudocercosporella herpotrichoides, is one of a number of stem-based pathogens. Detection of the pathogen by classical methods is difficult. Visual symptoms of the disease are easily confused with the superficial lesions caused by other less invasive stem-based fungi such as Pseudocercosporella anguioides, Fusarium avenaceaum, Microdochium (Fusarium) nivale, and Rhizoctonia cerealis. Plating out of infected tissue and isolation of the organism in pure cultures to confirm diagnosis is time consuming. It can take up to 6 weeks and requires considerable expertise. Prevention of the disease depends upon rapid application of the appropriate fungicide. Because of the long delay in confirming the presence of the pathogen farmers are generally advised to spray whenever the disease is suspected. However with lower profit margins farmers will be tempted to risk not spraying or using cheaper fungicides that will kill other pathogens but not P. herpotrichoides.

It would therefore be of considerable advantage if a rapid test method was available for distinguishing P. herpotrichoides from other fungal pathogens.

It would also be desirable to provide a way of speeding up of the time taken to screen all potential new fungicides for their effectiveness against P. herpotrichoides.

Recent times have seen rapid development and rapid progress in the development of immunological assay methods in the field of medical diagnosis. More recently, immunological methods have been employed in non-medical areas, and in particular in the preparation of assays for fungal pathogens. A review of immunological diagnostic assays for fungal plant pathogens was given by F. M. Dewey at the Brighton Crop Protection Conference - Pest and Diseases - 1988, and is reported in an associated paper.

As indicated in that paper, the development of immunological assays for the detection of fungi in diseased plants has been slow. There are a number of reasons for this, but an important reason is the difficulty in producing antisera (polyclonal antibodies) or monoclonal antibodies which are sufficiently specific to enable a test to be developed for a particular fungus, in view of the tendency of antisera or monoclonal antibodies to cross-react with other pathogens. The paper referred to above refers to the raising of antisera and the production of monoclonal antibodies to Pseudocercosporella species, employing as an immunogen surface washing from cultures of Pseudocercosporella anguioides. Hitherto however, antisera produced have been incapable of distinguishing between species of Pseudocercosporella, or distinguishing Pseudocercosporella from other stem-based pathogens in particular Microdochium (Fusarium) nivale.

In unpublished work, the present inventors have carried out extensive investigations in order to raise a species-specific antiserum and monoclonal antibodies capable of being used in an assay to distinguish Pseudocercosporella species. Those investigations have indicated that, contrary to expectation antibodies with sufficiently high specificity cannot be obtained by employing as an immunogen

a) cell-free washings from culture material
b) mycelial fragments (with and without Freund's adjuvant)
c) protein precipitates from culture fluids
d) carbohydrate precipitates from culture fluids.

In particular, none of the antibodies so far raised have been found to be sufficiently specific to P. herpotrichoides, and in particular sufficiently specific to distinguish eyespot lesions from those caused by M. nivale.

We have now discovered that species-specific antibodies against P. herpotrichoides may be prepared by using a particular immunogen in the raising of such antibodies. The immunogen of the present invention may be prepared using ammonium sulphate precipitation, and is believed to be a proteinaceous material or glycoprotein having a molecular weight in the range 50 to 300 KD, predominantly 75 to 120 KD, mainly about 83 KD. When inoculated into an immunocompetent host in favourable circumstances, the immunogen of the present invention is successful in causing the generation of antibodies which are able to distinguish P. herpotrichoides from Rhizoctonia cerealis, Fusarium culmorum, F. avenaceum, and M. nivale.

In accordance with the present invention, there is provided an immunogenic material for raising antibodies to Pseudocercosporella herpotrichoides, which material consists essentially of P. herpotrichoides-derived proteinaceous material or glycoprotein which is substantially free of material having a molecular weight of less than 50 KD and of material having a molecular weight of greater than 300 KD. The material preferably contains predominantly material having a molecular weight in the range 75 to 120 KD, and in particular, predominantly material having a molecular weight of about 83 KD.

The invention also provides an immunogenic material for raising antibodies to Pseudocercosporella herpotrichoides (P. herpotrichoides), which material comprises P. herpotrichoides-derived material from which has been removed,

a) the material which is caused to precipitate by the presence in an aqueous solution thereof of 30 weight percent $(NH_4)_2SO_4$, and

b) the material which is not caused to precipitate by the presence in an aqueous solution thereof of 70 weight percent $(NH_4)_2SO_4$.

The technique of ammonium sulphate precipitation in the purification of protein is one which has been very well known for many years. It is described, for example, in "PROTEIN PURIFICATION, PRINCIPLES AND PRACTICE" (Robert Scopes, published by Springer-Verlag). As the concentration of ammonium sulphate in a protein-containing solution is increased, proteins of progressively lower molecular weight are precipitated.

For the purposes of the present invention, the fraction of the antigenic material which is used to raise antibodies is that fraction from which material has been removed which is precipitated by thirty percent ammonium sulphate. Similarly, that fraction is removed which is not precipitated by seventy percent ammonium sulphate. Although the preferred way of removing the materials indicated is by actually carrying out an ammonium sulphate precipitation, clearly any other separation method which results in the same material being removed may alternatively be employed. The fraction obtained when such an ammonium sulphate precipitation is carried out is known as the 30s - 70s fraction. Surprisingly, it has been found that a very much more specific immune response can be obtained by employing such an immunogen in an immunisation procedure, as compared with the immune response which is obtained either using the antigenic material without treatment, or after other forms of treatment. In particular, antibodies can be raised which are able to distinguish <u>Pseudocercosporella herpotrichoides</u> from other species with which cross reaction normally is observed.

A further aspect of the invention provides a process for raising antibodies to <u>P</u>. <u>herpotrichoides</u>, comprising immunising an animal, for example, a mouse, with the above-described antigenic material. Also within the scope of the invention are the resulting antibodies obtained. Furthermore, the antigenic material in accordance with the invention may be employed in the production of hybridoma cells capable of producing monoclonal antibodies, by immunising an animal, such as a mouse, with the antigenic material to produce immunocompetent cells, and fusing such cells with myeloma cells to produce an immunocompetent hybridoma. Also within the scope of this invention are monoclonal antibodies produced by culturing such hybridoma cells.

Both polyclonal antisera and monoclonal antibodies in accordance with the invention may be employed in immunoassays for <u>Pseudocercosporella</u> <u>herpotrichoides</u>. In general, an immunoassay will involve carrying out a specific binding reaction between the antibody and <u>P</u>. <u>herpotrichoides</u>-derived material which it is desired to assay. The specific binding reaction is carried out in such a way as to produce a detectable change, which is dependent upon the amount of <u>P</u>. <u>herpotrichoides</u>-derived material in the sample. In a preferred immunoassay method, the specific binding reaction is carried out so as to bind to a solid support an amount of a detectable label which is dependent upon the amount of <u>P</u>. <u>herpotrichoides</u>-derived material in the sample. The method may be a so called "sandwich" or "competition" assay, as is well known in the art. The detectable label may be, for example, a radioisotope, or a fluorescent label, but in a preferred embodiment is an enzyme such as an oxidase, which can be determined quantitatively or qualitatively by known development reactions, for example, to produce a colour change.

In a preferred embodiment, a monoclonal antibody produced in accordance with the invention is coated non-specifically on to a micro-titre well, subsequently reacted with the sample material and washed. The bound sample is then treated with a further secondary antibody against the eyespot pathogen <u>P</u>. <u>herpotrichoides</u>, which may be a polyclonal antibody or a second monoclonal. The secondary antibody is preferably an antibody in accordance with the invention but alternatives may be an anti <u>P</u>. <u>herpotrichoides</u> antibody which is not obtained using an antigen in accordance with the invention. An antibody enzyme conjugate capable of recognising the polyclonal antibody or second monoclonal is then introduced, thereby to immobilise to the solid wall of the micro-titre plate an amount of the enzyme which is dependent upon the amount of antigenic material in the sample.

Alternatively, an enzyme or other suitable detectable label may be conjugated directly to the secondary enzyme.

The present preferred protocol for carrying out a "sandwich" enzyme-linked immunoassay is as follows, using 50µl sample volumes in 300µl sample wells.

1. Coat wells with culture supernatant containing monoclonal antibody (50µl per well). Leave overnight at 4°C.

2. Wash wells with phosphate-buffered saline containing 0.05% surfactant (Trade Mark Tween 20) (referred to hereafter as PBST). 4 x 5 minute washes, invert and flick out remaining liquid between washes and after final wash.

3. Block pre-coated wells for 10 minutes with 1% bovine serum albumen (BSA) in phosphate buffered saline.

4. Wash (4 x 5 minutes with PBST) as in step 2.

5. Add 50μl of sample extract to each well. Samples are prepared in 0.1M acetate buffer pH 4.8 (1/5 w/v)

6. Incubate samples for one hour at ambient temperature.

7. Wash (4 x 5 minutes with PBST) as in step 2.

8. Place 50μl of rabbit anti-serum freshly diluted 1 in 500 into 0.1M acetate buffer pH 4.8 in each well. Incubate at ambient temperature for one hour.

9. Wash (4 x 5 minutes with PBST) as in step 2.

10. Add 50μl of anti-rabbit peroxidase conjugate (SIGMA A 4914), freshly diluted into PBST 1 in 5000. Incubate at ambient temperature for one hour.

11. Wash (4 x 5 minutes) with PBST as in step 2.

12. Add to each well 50μl of the substrate in a citrate/acetate buffer (pH 5.4) containing 100mg/l tetramethyl benzidine, and 500μl/l 308 $H_2O_2$

13. Stop reaction by adding 50μl of 3N $H_2SO_4$ to each well.

14. Read Absorbance at 450nm. Use healthy control samples as control blanks.

In an alternative embodiment, an agglutination assay, which may be of generally conventional form, may be carried out for the P. herpotrichoides antigen. For example, antibodies in accordance with the invention may be bound non-specifically to killed cells of Staphylococcus aureus, which are then exposed to the antigen. Because the antigen is multivalent, agglutination of the cells takes place, which can be made visible to the eye by the addition of a suitable dyestuff, e.g., basic fuchsin.

The novel antibodies in accordance with the invention may be incorporated into an immunoassay test kit, comprising the various reagents components and reagents necessary for carrying an immunoassay for P. herpotrichoides, in a suitable form. Such a kit may be adapted specifically for carrying out an immunoassay of the kind just described, or of any known and conventional type, but is characterised in that the novel antibody of the invention is employed.

A number of preferred embodiments of the present invention are described in the following examples.

Example 1

Preparation of antigenic material.

Immunogen was prepared from extracts of a freeze-dried culture of a wheat-pathotype MBC sensitive isolate of Pseudocercosporella herpotrichoides. The isolate was originally obtained from Rothamsted Experimental Station and designated BY151.

The pathogen was grown axenically on an autoclaved potato-dextrose broth in petri-dishes. The medium was inoculated with three six millimetre plugs taken from the advancing edge of a two week old solid plate culture of BY151 isolate grown on Oxoid potato dextrose agar (39 g/l). Inoculated petri-dishes were incubated in polythene bags (three plates per bag) at 15°C, in near W black light. After incubation for ten days, the mycelium was separated from the culture medium by centrifuging at 2000g for five minutes. The supernatant was decanted off, and the mycelium immediately frozen in liquid nitrogen, freeze dried, and stored at -20°C until required.

Mycelial extracts were prepared by suspending the mycelium in phosphate buffered saline (PBS) (NaCL, 0.8%; KCL, 0.02%; $Na_2HPO_4$, 0.115%; $KH_2PO_4$, 0.02%; pH 7.2), vortexing briefly and then centrifuging at 1300g for 2 minutes to remove the cell debris. The supernatant was removed and a saturated solution of ammonium sulphate added to give a 30% solution. The latter was left to stand for 16 hours at 4°C and then spun at 15000g for 30 minutes at 4°C to precipitate the insoluble material. The supernatant was decanted and subjected to further addition of saturated ammonium sulphate, as will be described in more detail below, to prepare the 30s-70s fraction.

The solid residue from the 30% ammonium sulphate precipitation was redissolved in a small volume of phosphate buffered saline and dialysed against 3 changes of 2 litres of phosphate buffer at 4°C for 24 hours, and then freeze dried. This material is referred to herein as the 30s fraction.

The supernatant from the 30% ammonium sulphate precipitation was subjected to the addition of further saturated ammonium sulphate to give a final concentration of 70%. This solution was mixed thoroughly by vortexing, and again left to stand for 16 hours at 4°C. The material precipitated at this concentration was collected by spinning at 15000g for 30 minutes at 4°C and the supernatant was removed by decanting. The precipitate was solubilized in a small volume of phosphate buffered saline and dialysed against 3 changes of 2 litres of phosphate buffer at 4°C for 24 hours, to produce an antigenic material, hereinafter referred to as the 30s-70s fraction. The 30s-70s fraction was freezed-dried and stored at -20°C until required.

### Example 2

### Raising of antibodies in mice

Three female Balb/c mice (approx. 6 weeks old) were given intraperitoneal injections at two week intervals. Each injection consisted of 300 μl of a PBS extract of the 30s-70s freeze dried immunogen (2 mg containing 31 μg of protein). Mice were bled from the tail vein after the 3rd, 5th and 7th injections. The spleen was removed from one mouse three days after the third injection and cells from this spleen used for the fusion from which the hybridoma cell line secreting the P. herpotrichoides-specific monoclonal antibody was derived by selecting those which did not cross-react with other fungi, by the method of Dewey et al. (Journal of General Microbiology, 135, 361-374, 1989).

### Example 3

### Raising antibodies in Rabbits

Three New Zealand white rabbits, between 1.5 and 2Kg in weight, two female one male, were given four sets of injections. The first three injections were given at weekly intervals and the fourth after a 6 week interval. Injections were given intramuscularly into the fore and hind limbs. Each set of injections contained a total of 2.2mg of the immunogen (i.e. approx. 600 μg protein) solubilized in PBS and emulsified for the first injection with Freund's complete adjuvant and with Freund's incomplete adjuvant for the subsequent injections. Test bleeds were done prior to immunization and 6 days after the third and fourth injections. The final bleed was made 8 days after the last injection. Antisera were prepared as described in Dewey et al. (1984), Phytopathology, 74, 291-296 and pooled after testing the titres by enzyme-linked immunoassay.

### Example 4

### Production of Hybridomas and Monoclonal Antibodies

Hybridoma cell-lines were raised by fusing splenocytes of immunized mice with myeloma cells in the presence of polyethylene glycol, Mw 1,500. Hybridoma supernatants were screened by enzyme-linked immunoassay against microtitre wells coated with cell-free antigens from freeze dried cultures of P. herpotrichoides solubilized in phosphate buffered saline (2 mg in 1 ml). Supernatants from positive cell-lines were further tested against antigens from freeze dried cultures of the other stem-based pathogens and Rhizoctonia solani. Promising cell-lines were re-cloned twice by limiting dilution, further tested and preserved in liquid nitrogen. Supernatants were stored at 4°C after the addition of sodium azide (final concentration 0.02%). Details of all the above methods were the same as those reported by Dewey et al. (Journal of General Microbiology, 135. 361-374, 1989).

Monoclonal antibodies were harvested by growing up the resulting cell-lines and collecting the supernatant by conventional methods.

### Example 5

### Enzyme-linked Sandwich Immunoassay

The immunoassay method was based on that developed by Clark and Adams (1977) Characteristics of the microtitre plate method of enzyme-linked immunosorbent assay for the detection of plant viruses. J. gen. Virology. 34, 475-483. Wells of a 96-well micro-titre plate were coated overnight with supernatants containing monoclonal antibodies prepared in accordance with Example 4.

The following day, the wells were washed four times with PBST and dried under a laminar flow hood. The wells were blocked by the addition of 1% bovine serum albumen in PBS for 10 minutes and then washed 4 times as before. Sample extracts were added, and the wells incubated to bind the sample material to the immobilised monoclonal antibodies. The wells were washed to remove unbound sample, and subsequently treated with a solution of a polyclonal anti-Pseudocercosporella herpotrichoides rabbit anti-serum prepared in accordance with Example 4, diluted one in 500 in PBST. Excess anti-serum was removed by washing.

The binding of the rabbit anti-sera was then detected by means of a commercial antibody-enzyme conjugate (Sigma A4914) raised in goats, which recognises the non-specific (Fc) region of rabbit antibodies.

The bound antibody enzyme-conjugate was then developed by reaction with tetra-methyl benzidine in dimethyl sulfoxide (see Dewey et al (1989) J. Gen. Microbiology 135, 361-364), to produce a coloured product. Absorption at 450nm was then measured spectrophotometrically, using a conventional plate reading machine. The results obtained for known sample types containing respectively P. herpotrichoides, M. nivale and R. cerealis are shown in Table 1.

## Table 1

| Extract from plant infected with: | Absorbance readings |
|---|---|
| Healthy control | 0.07 |
| P. herpotrichoides | 0.75 |
| M. nivale | 0.11 |
| R. cerealis | 0.15 |

It can be seen that the assay is easily able to distinguish between species of P. herpotrichoides and M. nivale and R. cerealis.

PBST = PBS plus 0.05% Tween 20. see Dewey et al. J. Gen. Microbiology 135 361-364.

Example 6

Enzyme-linked immunosorbent assay

Enzyme-linked immunosorbent assays were carried out to determine the specificity of the monoclonal antibody produced in accordance with Example 4 and the Rabbit (polyclonal) antiserum produced in accordance with Example 3 using antigen coated wells

The immunoassay method was based upon that developed by Dewey et al. (Journal of General Microbiology, 135, 361-374, 1989). The wells of a 96-well micro-titre plate were coated overnight with 50 μl of phosphate buffered saline extracts from freeze-dried cultures of Pseudocercosporella herpotrichoides (2 mg in 1 ml) or phosphate buffered saline, cell free, surface washings of 14-day old cultures of other pathogens as shown in Table 2 (1 ml per culture slant, further diluted into 20 ml). The following day the wells were washed 4 times with PBST and dried as in accordance with Example 5. Wells were subsequently incubated with either the monoclonal antibody of Example 4 or rabbit antiserum of Example 3 raised against the 30s-70s fraction diluted one in 500, 1000, 2000 or 4000 into PBST for 30 minutes.

The binding of the antibodies to the fungal antigens was detected by means of a commercial antibody-enzyme conjugate (Sigma A 0412 for murine antibodies, Sigma A 4914 for rabbit antibodies) in accordance with Example 5. The bound antibody enzyme conjugate was then developed by reaction with the substrate, tetramethyl benzidine, in accordance with Example 5. The results are presented in Table 2.

It can be seen that both the monoclonal antibody and the rabbit 30s-70s polyclonal antibody are specific, significant cross-reactions do not occur with unrelated fungi or, more importantly, with those fungi that are commonly found to invade the stem-base.

## Table 2

| Test fungi | Monoclonal | | Rabbit (polyclonal) s/w antiserum dilutions | | | |
|---|---|---|---|---|---|---|
| | s/w | f/d | 500 | 1000 | 2000 | 4000 |
| P. herpotrichoides | 0.33 | 0.43 | 1.31 | 1.25 | 1.23 | 1.20 |
| P. anguioides | 0.09 | | 1.33 | 1.23 | 1.28 | 1.33 |
| F. avenaceum | 0.06 | | 0.27 | 0.19 | 0.11 | 0.08 |
| F. culmorum | 0.03 | | 0.14 | 0.08 | 0.05 | 0.04 |
| M. nivale | 0.06 | 0.11 | 0.60 | 0.36 | 0.22 | 0.16 |
| R. cerealis | 0.09 | | 0.45 | 0.38 | 0.18 | 0.12 |
| Alternaria sp. | 0.12 | | 0.34 | 0.25 | 0.17 | 0.13 |
| R. solani | 0.11 | | n/d | n/d | n/d | n/d |
| P. waksmanii | 0.11 | | 0.51 | 0.29 | 0.17 | 0.13 |

Absorbance readings

f/d= tested against extracts from freeze dried mycelium
s/w= tested against surface washings

n/d  not determined.

Example 7

Enzyme-linked immunosorbent assay to demonstrate the greater specificity of polyclonal antisera raised against the 30s-70s fraction versus that raised against the 30s fraction.

Antisera were raised in mice and rabbits against the 30s-70s and 30s fractions and tested for their specificity against antigen coated wells by the method of Example 6. They were tested against surface antigens of Pseudocercosporella herpotrichoides Microdochium nivale and Rhizoctonia solani. The latter two fungi, had from previous studies, been found to be the fungi with which non-specific antisera most commonly cross-reacted. The results are presented in Table 3.

It can be seen that antisera raised against the 30s-70s fraction in both mice and rabbits has a very much higher specificity than that raised against the 30s fraction. These results accord well with those presented in Example 5 in which extracts from plants infected with the stem-based fungi were tested.

## Table 3

Note: all antisera were tested against microtitre wells coated with a cell-free phosphate buffered saline extract of freeze dried mycelium (2 mg/ml).

Reciprocal dilutions of antiserum

| | 6400 | 12800 | 25600 | 51200 | 102400 |
|---|---|---|---|---|---|
| **Rabbit 70s fraction** | | | | | |
| P. herpotrichoides | 1.22 | 1.07 | 0.85 | 0.61 | 0.37 |
| M. nivale | 0.53 | 0.34 | 0.21 | 0.13 | 0.10 |
| **Mouse 70s fractions** | | | | | |
| P. herpotrichoides | 1.14 | 0.93 | 0.66 | 0.52 | 0.34 |
| M. nivale | 0.78 | 0.58 | 0.31 | 0.20 | 0.13 |
| R. solani | 0.60 | 0.40 | 0.24 | 0.16 | 0.12 |
| **Mouse 30s fraction** | | | | | |
| P. herpotrichoides | 0.87 | 0.71 | 0.45 | 0.30 | 0.22 |
| M. nivale | 0.82 | 0.59 | 0.40 | 0.28 | 0.19 |
| R. solani | 0.57 | 0.43 | 0.39 | 0.19 | 0.15 |

Example 8

Characterisation of the antigen by Gel electrophoresis and Western Blotting

Gel electrophoresis and Western Blotting using gels of different strengths were carried out to determine the molecular weight and nature of the antigen recognized by the specific monoclonal antibody in extracts from freeze dried samples of P. herpotrichoides and the 30-70s fraction and to demonstrate that the same antigen is recognized in extracts from infected but not healthy plant material.

Gel electrophoresis was carried out using the high pH, non-dissociating, discontinuous buffer system described by Hames (1990) in "GEL ELECTROPHORESIS OF PROTEINS, A PRACTICAL APPROACH" (B.D. Hames and D. Rickwood, published by IRL Press at Oxford University Press). Non-dissociating, discontinuous slab gels comprising 5%, 5,5%, 6%, 7%, 8%, 9% and 10%, main gel with a 3.75% stacking gel were run at a constant voltage of 250v for three hours.

Samples for electrophoresis were prepared by extracting 1g of plant material in 1ml of sample buffer [0.0625 M Tris-HCl pH 6.8, 10% (w/v) sucrose and 0.002% (w/v) bromophenol blue], centrifuging at 13000g for 2 minutes to remove debris and loading sample volumes of 50µl/lane onto the gels.

After electrophoresis, Western Blotting was carried out by the following method. Proteins from the non-dissociating gels were transferred electophoretically using a semi-dry blotter (JKA-BIOTECH, Denmark) onto a polyvinylidene difluoride membrane (trade mark IMMOBILON-P) at 0.8 mA/cm² of membrane for 1 hour with the following buffers: anode buffer 1, 0.3 M Tris in 20% (v/v) methanol, pH 10.4 anode buffer 2, 25mM Tris in 20% v/v methanol, pH 10.4 and cathode buffer, 40mM 6-amino-n-hexanoic acid in 20% (v/v) methanol, pH 7.2. The membrane was initially wetted in methanol and then water. After transfer, membranes were blocked with a 5% solution of bovine serum albumin (SIGMA-7030) in Tris-buffered saline (TBS) [20mM Tris-HCl pH 8.2, 0.9% (w/v) NaCl, 20mM sodium azide] for 1 hour before immunostaining. The membranes were washed 3 times for 5 minutes each washing, the TBS containing 0.1% (w/v) bovine serum albumen. The membranes were then incubated for 16 hours in P. herpotrichoides specific monoclonal antibody (undiluted hybridoma culture supernatant) and again washed (3 x 5 minute washes) with TBS containing 0.1% (w/v) bovine serum albumen. They were then incubated for a further 16 hours in goat anti-mouse 1gG +1gM (H+L) gold conjugate (BL grade produced by Amersham International) diluted 1/100 into TBS + 0.1% (w/v) bovine serum albumen.

The membranes were again washed twice (5 minutes) with TBS containing 0.1% (w/v) bovine serum albumen, and finally twice (1 minute) with distilled water. The gold stain was enhanced using the Amersham intenSE BL silver enhancement kit. Membranes were agitated gently for all incubation.

The major protein/glycoprotein band seen in the 30s-70s fraction, run on different gels, was calculated to have a molecular ratio of approximately 83kD. This band can also be seen in extracts of whole freeze dried mycelia. In a Western Blot of a 7% gel, the major protein/glycoprotein band can be seen to be stained positively using the specific monoclonal antibody, it can be seen in both whole extracts from the freeze dried material, in the 30s-70s fraction and in extracts from infected but not healthy plant material.

## Claims

1. An immunogenic material for raising antibodies to Pseudocercosporella herpotrichoides, which material consists essentially of P. herpotrichoides-derived proteinaceous material or glycoprotein which is substantially free of material having a molecular weight of less than 50 KD and of material having a molecular weight of greater than 300 KD.

2. An immunogenic material for raising antibodies to Pseudocercosporella herpotrichoides, which material comprises P. herpotrichoides-derived material from which has been removed,
   a) the material which is caused to precipitate by the presence in an aqueous solution thereof of 30 weight percent $(NH_4)_2SO_4$, and
   b) the molecular weight fraction which is not caused to precipitate by the presence in an aqueous solution thereof of 70 weight percent $(NH_4)_2SO_4$.

3. A process for raising antibodies to P. herpotrichoides, which process comprising immunizing an immunocompetent animal with an immunogenic material as claimed in Claim 1 or Claim 2, and collecting antiserum produced by the animal.

4. A process as claimed in Claim 3, wherein the animal is a mouse or rabbit.

5. Antiserum prepared by immunising an animal with an immunogenic material as claimed in Claim 1 or 2, and harvesting the resulting antiserum.

6. A hybridoma produced by immunising an animal with an immunogenic material as claimed in Claim 1 or Claim 2 to produce immunocompetent cells, fusing the immunocompetent cells produced with a myeloma to produce a hybridoma, and selecting at least one hybridoma cell line producing monoclonal antibodies which do not cross react with fungi other than Pseudocercosporella herpotrichoides.

7. Species-specific monoclonal antibodies against Pseudocercosporella herpotrichoides, Produced by culturing a hybridoma in accordance with Claim 5, and harvesting the antibodies produced.

8. A method of carrying out a species-specific immunoassay for P. herpotrichoides, which process comprises carrying out a specific binding reaction between antiserum in accordance with Claim 5 or a monoclonal antibody in accordance with Claim 7, and P. herpotrichoides-derived material which it is desired to assay, and thereby producing a detectable change dependent upon the amount of P. herpotrichoides-derived material in the sample.

9. A method as claimed in Claim 8, wherein an amount of a detectable label is bound to a solid support, the amount of the detectable label which is bound being dependent upon the amount of P. herpotrichoides-derived material in the sample.

10. A method as claimed in Claim 8 which is an agglutination assay, wherein the rate of agglutination of a material varies in dependence upon the amount of P. herpotrichoides-derived material in the sample.

11. A test kit for carrying out an immunoassay for P. herpotrichoides-derived material, characterised in that the test kit comprises an antibody as claimed in Claim 7.